# EUROPEAN PATENT SPECIFICATION

(11) **EP 1 991 128 B1**
(45) Date of publication and mention of the grant of the patent: **11.04.2012**
(21) Application number: 06799606.6
(22) Date of filing: 16.10.2006
(51) Int. Cl.: A61B 7/00

(54) **METHOD, DEVICE AND SYSTEM FOR CARDIO-ACOUSTIC SIGNAL ANALYSIS**
VERFAHREN, VORRICHTUNG UND SYSTEM FÜR DIE KARDIO-AKUSTISCHE SIGNALANALYSE
PROCEDE, DISPOSITIF ET SYSTEME POUR ANALYSE CARDIO-ACOUSTIQUE DE SIGNAUX

(30) Priority: 14.10.2005 EP 05077367
(43) Date of publication of application: 19.11.2008
(73) Proprietor: Medicalgorithmics Sp. Z.O.O., 02-590 Warszawa (PL)
(72) Inventor: DZIUBINSKI, Marek, PL-01-361 Warszawa (PL)
(74) Representative: Murphy, Colm Damien
(86) International application number: PCT/PL2006/000067
(87) International publication number: WO 2007/043902

(56) References cited:
- WO-A-93/22970
- US-A- 5 012 815
- BRUSCO M ET AL: "Digital phonocardiography: a PDA-based approach" CONFERENCE PROCEEDINGS. 26TH ANNUAL INTERNATIONAL CONFERENCE OF THE IEEE ENGINEERING IN MEDICINE AND BIOLOGY SOCIETY (IEEE CAT. NO.04CH37558) IEEE PISCATAWAY, NJ, USA, vol. 3, 2004, pages 2299-2302 Vol., XP010775437 ISBN: 0-7803-8439-3
- ZWICKER E ET AL: "AUDIO ENGINEERING AND PSYCHOACOUSTICS: MATCHING SIGNALS TO THE FINAL RECEIVER, THE HUMAN AUDITORY SYSTEM" JOURNAL OF THE AUDIO ENGINEERING SOCIETY, AUDIO ENGINEERING SOCIETY, NEW YORK, NY, US, vol. 39, no. 3, 1 March 1991 (1991-03-01), pages 115-126, XP000208423 ISSN: 1549-4950
- ERICKSON R ET AL: "In-vitro study of mechanical heart valve sound loudness as measured by ISO-532/B" PROCEEDINGS 1994 IEEE SEVENTH SYMPOSIUM ON COMPUTER-BASED MEDICAL SYSTEMS (CAT. NO.94CH3426-4) IEEE COMPUT. SOC. PRESS LOS ALAMITOS, CA, USA, 1994, pages 53-54, XP000499316 ISBN: 0-8186-6256-5

## Description

The invention relates to the field of visualization and automated analysis of the digitized cardiac auscultation (acoustic) signals.

Heart auscultation is a very old technique, which is still commonly used during medical examinations carried out by physicians.. It is performed using a standard non-electronic stethoscope, anelectronic analogue stethoscope or a modern digital stethoscope. Cardiac auscultation is based on auditory perception of sounds produced by the heart in certain areas of the human body. These sounds are often accompanied by special kind of maneuvers, which enhance some significant sounds, clicks or murmurs produced by the heart muscle and the blood flow across heart valves. Cardiac auscultation is difficult to perform properly due to the limitations of human hearing. This examination method requires nearly perfect hearing and extensive experience of the physician in dealing with rare cases of heart abnormalities. However, while experience increases with age, hearing deteriorates. Furthermore, acoustic signals have a very small amplitude and heart's activity is usually represented by very low frequencies, which remain on the edge of human perception. Also, pathologies detectable via cardiac auscultation are rare, and therefore medical doctors are not able to compare various cases representative of a given pathology. This makes the diagnosis even more difficult. Additionally, interpretation of the results of an auscultation exam is very subjective, because different doctors have different experience and hearing abilities. For this reason, despite its potential, cardiac auscultation is treated as a preliminary overview of the patient's heart condition, rather than a source of reliable information for potential treatment. Document US 5012051 A1 discloses a phonocardiograph.

Phonocardiography is the best known technology, which aids physicians in dealing with heart sounds. It is based on visualization of the time domain acoustic cardiac signal. The general idea of this technology has been adopted from the ECG analysis, where the time domain electrophysiological waveform is analyzed by a medical specialist. However such approach is not appropriate for acoustic signals, because listening to the signal differs from viewing the time domain waveform, especially since acoustic events may happen simultaneously at different frequencies. Phonocardiography may be improved by multi-band analysis, where several waveforms related to specific sub-bands are filtered out and often processed non-linearly. Such improvements allow to identify acoustic events related to different frequencies. However, this approach is still insufficient due to nonlinear complexity of sound perception. Alternative methods of cardiac auscultation signal processing are based on simulating human auditory system. In those methods the time domain waveform is represented as a 3 dimensional signal (digital image - time-frequency-amplitude map) where changes of the amplitude over time, for certain frequencies can be observed. Human auditory system functions in a similar manner, i.e. a listener detects energy of certain acoustic events and is able to determine their frequency and the moment of their occurrence (i.e. as an example: one can notice that at certain time high frequency click occurred and after some time low frequency clatter was present, etc.). However, these methods do not accurately represent the human auditory system and therefore do not properly show what should be heard (but still are closer to simulating human auditory system than phonocardiography). It is due to the fact that human auditory system operates with specific frequency resolution, which is very high for lower frequencies and decreases with the increase of the sounds frequency. Additionally, some acoustic events are inaudible, when they occur in direct presence (in time or frequency) of louder events (this phenomenon is known as perceptual masking). Furthermore, loudness of certain acoustic events is not directly related to their amplitude (energy), i.e. some frequencies are heard better than others.

In general the methods described above are based on transformations of the time domain signal into the frequency representation. They are usually based on methods such as FFT (Fast Fourier Transform - Fourier spectrum), DWT (Discrete Wavelet Transform - wavelet spectrum), CWT and several other techniques with some nonlinear enhancements, which however, do not take into account complexity of the human auditory system.

A standard cardiac stethoscope examination enables a doctor to detect heart dysfunctions related to blood flow through the heart, such as heart valves dysfunctions - valves leakiness (i.e. regurgitation) or improper (not complete) opening of the valve (i.e. stenosis). Other common dysfunctions are valve calcification or other physiological heart defects. Cardiac auscultation is also commonly used to monitor the performance of artificial (implanted) mechanical valves, which tend to seal after some time and may require replacement.

The so called heart ECHO (USG of the heart) is an alternative technology (alternative examination method), which allows to precisely evaluate valve performance and detect other pathologies of the heart muscle. This method is however very expensive or has limited accessibility. Therefore, it is very important to improve the standard cardiac auscultation technology and develop an inexpensive and common alternative to the cardiac stethoscope examination.

It is an object of the invention to provide a method, device and system for cardio-acoustic signal analysis that takes into account characteristic of human auditory system.

The invented method comprises the steps of receiving an acoustic sensor signal representative of heart sounds, representing the acoustic sensor signal in a time-frequency-amplitude domain, applying a psycho-acoustic model to the acoustic sensor signal in the time-frequency-amplitude domain in order to generate a representation of the acoustic sensor signal in a time - perceptual frequency - perceptual loudness domain. The method preferably further comprises the step of simulating periodicity perception. It is also advantageous when the method also comprises the step of automatic interpretation of the of the representation of the acoustic sensor signal in a time - perceptual frequency - perceptual loudness domain.

A device according to the invention comprises input unit for receiving an acoustic sensor signal representative of heart sounds, means for representing the acoustic sensor signal in a time-frequency-loudness domain, means for applying a psycho-acoustic model to the acoustic sensor signal in the time-frequency-amplitude domain, output unit for presenting the acoustic sensor signal in a time - perceptual frequency - perceptual loudness domain.

A system according to the invention comprises the device as described earlier as well as the means for transmitting the acoustic sensor signal remotely to the input unit, where the input unit is operative to receiving the acoustic sensor signal remotely.

Additionally, the inventions preferably further comprise means for simulating periodicity perception of the acoustic events, i.e. humans have the ability to concentrate their attention on repetitive events (rhythmic events such as heart beat), rejecting incidental events, which usually are parasite disturbances.

Furthermore, the inventions preferably comprise means for automatic interpretation of the representation of the acoustic sensor signal in a time - perceptual frequency - perceptual loudness domain.

In general, psychoacoustics (human auditory system modelling) is based on several observations: humans perceive sounds in terms of their amplitudes, frequencies and time localization. Sounds of the same amplitudes, but different frequencies may be perceived as differently loud. Amplitude differences between sounds are frequency dependent, i.e. loudness relation between sounds of different frequencies varies with accordance to their energy. Quiet sounds may be masked (made inaudible) by louder sounds (maskers). This phenomenon depends on time and frequency distance between masked sounds and maskers. Perception of frequencies is non linear, i.e. resolution of human auditory system is much higher for lower sounds, than for higher sounds. Noisy (chaotic) and periodic (tonal) components of the sounds are perceived differently. Humans, while listening to the heart auscultation sounds, are able to focus on repetitive events, while some random events may be "separated" and disregarded.This innovation is based on a method of transforming the time domain waveform into the time - perceptual frequency - perceptual loudness domain. Such representation is obtained with regard to the mathematical model of the human auditory system. Additionally the innovation contains a set of algorithms responsible for simulating periodicity perception (pattern tracking) of acoustic events, i.e. humans are able to concentrate their attention on repetitive events (rhythmic events such as heart beat), rejecting incidental events, which usually are parasite disturbances.

Further, based on a periodicity analysis (pattern tracking and pattern tracking recovery), the auscultation signal may be significantly enhanced. Disturbing noise and parasite impulsive clicks of non-repetitive type may be removed, resulting in restored time domain representation of the auscultation sound.

The invention is well illustrated by the accompanying figures. The drawings relate to an embodiment of the invention and are an example of putting the invention into practice, therefore, they should not be interpreted as limiting the scope of the protection claimed.
Fig. 1 is a flow-chart of a preferred embodiment of the cardio-acoustic signal analysis algorithm.
Fig. 2 schematically illustrates an example of the device for the cardio-acoustic signal analysis.
Fig. 3 schematically illustrates an example of the system for the cardio-acoustic signal analysis.
Fig. 4 shows an example of the time - perceptual frequency - perceptual loudness domain representation of a cardio-acoustic signal.
Fig. 5 represents simulation of the ability of the human listener, with the use of the same signal as for Fig. 4.
Fig. 6 illustrates an example the auscultatory findings detected by the method according to the invention.

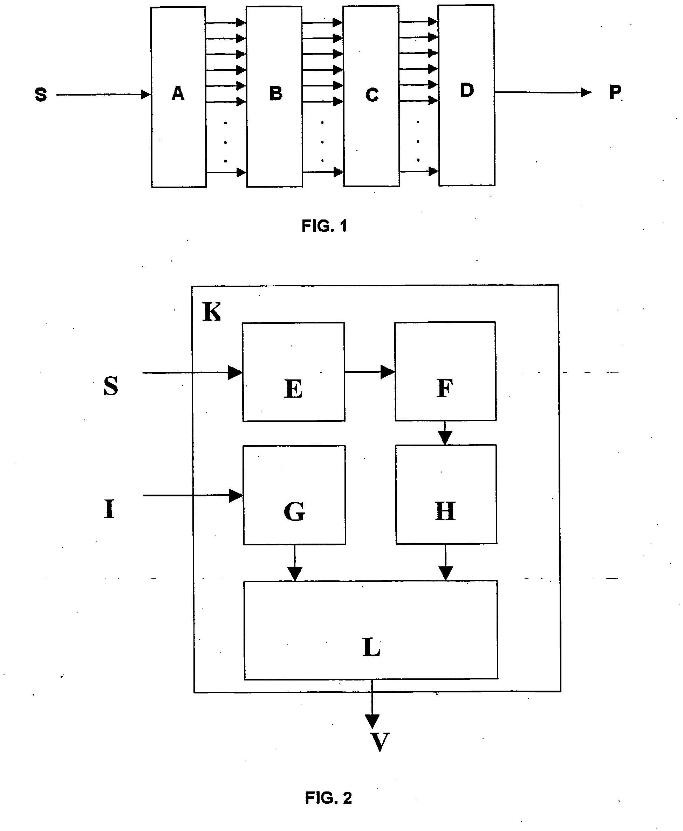

In Fig. 1., being a flow-chart of a preferred embodiment of the cardio-acoustic signal analysis algorithm, S represents a digital input acoustic sensor signal representative of heart sounds (time domain waveform), which is stored in an appropriate matrix and used as an input to block A. In block A the digital input acoustic sensor signal is subject to segmentation. i.e. the digital input acoustic sensor signal is divided into shorter blocks of equal length. Matrix of the time domain short blocks becomes input signal of block B. In block B each time domain segment is transformed into psychoacoustic spectral representation. Such representation is calculated with the use of inner products operations with regard to Bark units, i.e. frequency domain is represented by equally spaced spectral bins, however equal spacing refers to Bark units, thus such approach significantly differs from standard FFT, DWT, CWT, etc. The resulting representation vectors (each representing the input time domain blocks) is stored in an appropriate matrix and becomes input of block C, which is responsible for simulating Basiliar membrane excitation and simultaneous masking phenomenon, i.e. total excitation is constructed of very precise modeling of particular the membrane excitations. Matrix constructed with the calculated Basiliar membrane deformation vectors becomes input block D. Block D is responsible for simulating time domain Basiliar membrane behavior (time domain masking phenomenon), i.e. this method models the shape changes of the membrane over time. The output signal P from block D is a matrix built from Basiliar membrane shapes, thus P is a psychoacoustic representation (in the form of the 3D map) in time-perceptual frequency-perceptual loudness domain.

Fig. 2 represents schematically an embodiment of a device for the cardio-acoustic signal analysis, where such device may be for example incorporated into a microprocessor K. A signal storage and pre-processing block E receives a digital input acoustic sensor signal S representative of heart sounds (in time domain waveform). This block (E) is responsible for selecting a fragment of the input signal of 10 seconds duration. The chosen fragment is the most periodic part of the input signal, while periodicity indicator is obtained with the use of cross correlation algorithm applied for RMS envelope of the digital input acoustic sensor signal S. The next block of the device of the embodiment - the block F, is responsible for calculating psychoacoustic representation (in time-perceptual frequency-perceptual loudness domain), as described in detail in Fig. 1. Output signal from block F is further processed in block H, being a cardiac auscultation expert system (where parameterization of the examination information takes place). Simultaneously, a block G, being a periodicity detector (simulating human perception of repetitive events versus incidental events), is responsible for processing of examination information I (i.e. body position, auscultation area, inhalation/exhalation). Such information is considered in determining significance of certain cardiac events, such as clicks and murmurs, which may be increased or decreased, depending on the body position, exhalation or inhalation or may not be present for certain auscultation areas). Output signals from the block H and the block G are finally being processed in the block L, being a cardiac events detecting block. After such processing, an output signal V of the block L represents final results in the form of ascultatory findings.

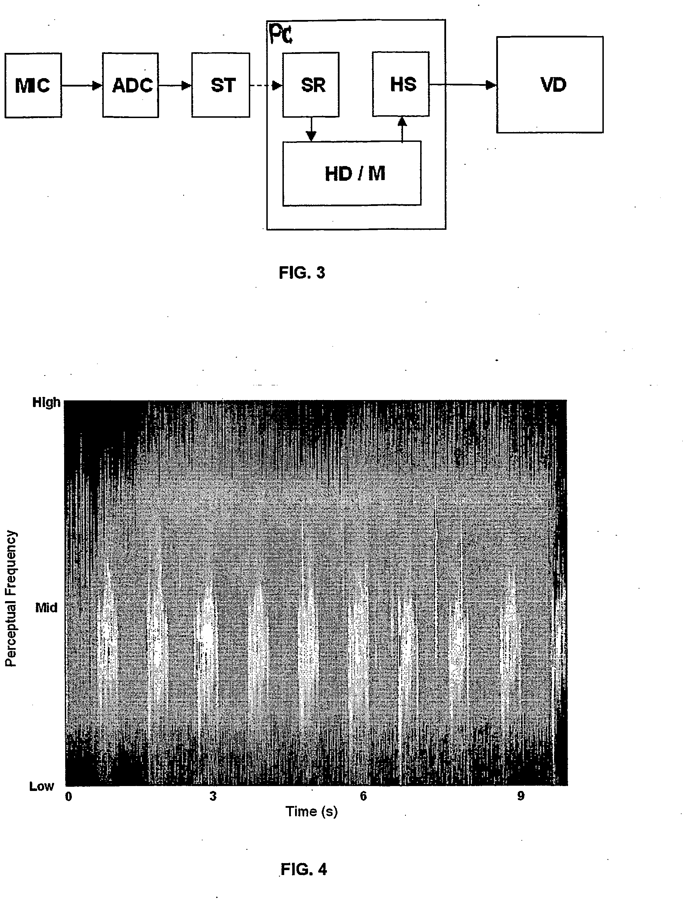

Fig. 3 schematically illustrates an embodiment of a system for the cardio-acoustic signal analysis, where MIC denotes a microphone, responsible for converting acoustic pressure changes into voltage changes. Further ADC represents an analog to digital converter (with antialiasing filter). Is function is to convert the analogue (voltage changes signal) into equally spaced digital signal. A signal radio/wire transmitter ST is responsible for sending the digital signal to a processing unit (e.g. a computer). Transmission could be carried out by USB cable, wireless transmitter, etc. PC is the processing unit (e.g. PC, PDA, device based DSP chip, etc). The processing unit comprises a signal receiver SR (e.g. USB adapter in case of USB connection, or radio receiver in case of radio transmission, etc.). The processing unit PC further comprises HS unit (e.g. a microprocessor) as described in more detail in Fig. 2. The processing unit PC also comprises data storage device HD/M, e.g. HD, RAM, etc. The last part of the embodiment of the system for the cardio-acoustic signal analysis is the so-called visualization unit VD (e.g. standard monitor, LCD display, etc.), which is responsible for enabling data presentation to a user.

Fig. 4. shows an example of the time - perceptual frequency - perceptual loudness domain representation, i.e. bassiliar membrane shape changes over time. In the representation of an certain cardio-acoustic signal, impulsive events (such as heart beats or clicks) are visible as vertical shapes of short duration, while long lasting murmurs are visible as frequency limited with relatively high center frequency shapes of longer duration.

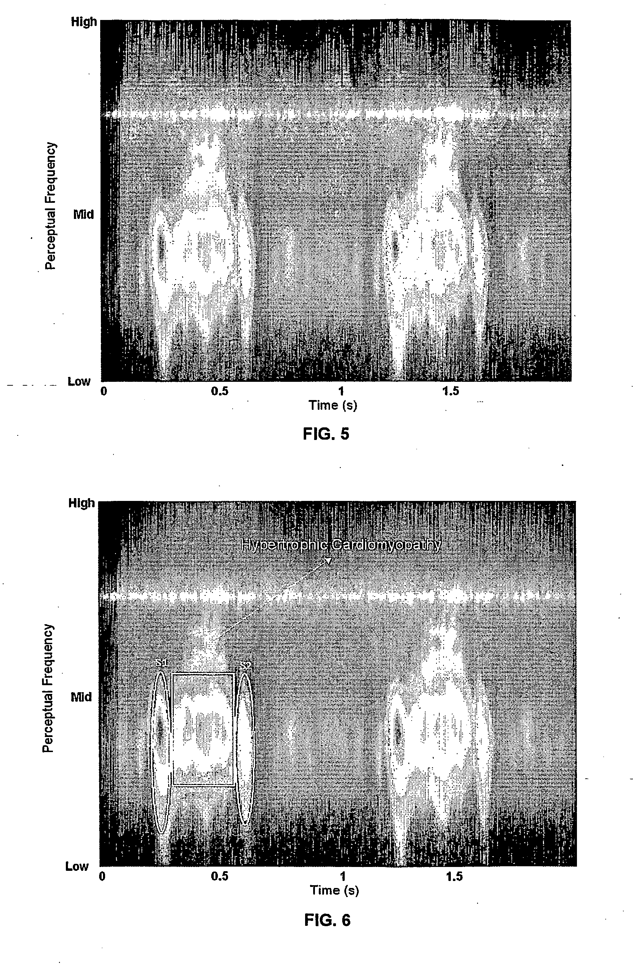

Fig. 5 represents simulation of the pattern tracking ability of the human listener, with the use of the same cardio-acoustic signal as used in Fig. 4. It can be observed that the representation is much smoother than the one showed on Fig. 4. with repetitive events emphasized.

Fig. 6. illustrates an example of the auscultatory findings detected in the signal shown in Fig. 5. In this example, the cardio-acoustic signal may be interpreted as indicative hypertrophic cardiomyopathy.

The invention represent a general concept of the use of human auditory system modeling application for analysis of auscultation acoustic signal, which could be implemented on various computers or other devices, with regard to specific requirements and knowledge of the users. The innovation is based on the algorithm (could be viewed as a DSP hardware device with dedicated DSP chip), which transforms the time domain waveform into the time-perceptual frequency-perceptual loudness domain.

Selected advantages of the invention:
- visualization of the signal exactly as it should be heard by an ideal listener;
- allows to utilize knowledge directly related to cardiac auscultation, gathered for last 200 years by thousands of doctors;
- objective analysis, i.e. the same signal always will result in the same representation and interpretation, which is not always possible if the same signal is heard by different people (since they may have different hearing abilities and listening experience);
- objective analysis of the artificial valve's performance, where ECHO performs poorly and is not very reliable.

## Claims

1. A method for cardio-acoustic signal analysis comprising the steps of:
receiving an acoustic sensor signal representative of heart sounds;
representing the acoustic sensor signal in a time-frequency-amplitude domain;
**characterized in that** the method further comprises the step of applying a psycho-acoustic model to the acoustic sensor signal in the time-frequency-amplitude domain in order to generate a representation of the acoustic sensor signal in a time - perceptual frequency - perceptual loudness domain.

2. A method as claimed in claim 1 **characterized in that** the method further comprises the step of simulating periodicity perception.

3. A method as claimed in claim 1 or 2 **characterized in that** the method further comprises the step of automatic interpretation of the of the representation of the acoustic sensor signal in a time - perceptual frequency - perceptual loudness domain.

4. A device for cardio-acoustic signal analysis comprising:
input unit for receiving an acoustic sensor signal representative of heart sounds;
means for representing the acoustic sensor signal in a time-frequency- amplitude domain;
**characterized in that** the device further comprises:
means for applying a psycho-acoustic model to the acoustic sensor signal in the time-frequency-amplitude domain;
output unit for presenting the acoustic sensor signal in a time - perceptual frequency - perceptual loudness domain.

5. A device as claimed in claim 4 **characterized in that** the device further comprises means for simulating periodicity perception.

6. A device as claimed in claim 4 or 5 **characterized in that** the device further comprises means for automatic interpretation of the of the representation of the acoustic sensor signal in a time - perceptual frequency - perceptual loudness domain.

7. A system for cardio-acoustic signal analysis comprising the device of any of claims 4 to 6, further comprising:
means for collecting an acoustic sensor signal representative of heart sounds; and
means for transmitting the acoustic sensor signal to the input unit.

8. A system as claimed in claim 7 **characterized in that** the means for transmitting the acoustic sensor signal are operative of remote transmission, where the input unit is operative to receiving the acoustic sensor signal remotely.

## Patentansprüche

1. Eine Methode für die kardio-akustische Signalanalyse, bestehend aus den folgenden Schritten:
Empfang eines akustischen Sensorsignals, das Herztöne darstellt;
Darstellung des akustischen Sensorsignals in einer Zeit-Frequenz-Amplitudendomäne;
**dadurch gekennzeichnet, dass** die Methode zusätzlich ein psycho-akustisches Modell an das akustische Sensorsignal in der Zeit-Frequenz-Amplitudendomäne ansetzt, um eine Darstellung des akustischen Sensorsignals im Bereich Zeit - wahrgenommene Frequenz - wahrgenommene Lautheit zu erzeugen.

2. Eine Methode gemäß Anspruch 1, **dadurch gekennzeichnet, dass** sie zusätzlich die Simulierung der Periodizitätswahrnehmung ermöglicht.

3. Eine Methode gemäß Anspruch 1 oder 2, **dadurch gekennzeichnet, dass** sie zusätzlich die automatische Interpretation der Darstellung des akustischen Sensorsignals im Bereich Zeit - wahrgenommene Frequenz - wahrgenommene Lautheit ermöglicht.

4. Eine Vorrichtung für die kardio-akustische Signalanalyse, bestehend aus:
Aufnahmegerät zur Aufnahme eines akustischen Sensorsignals, das Herztöne darstellt;
Mittel zur Darstellung des akustischen Sensorsignals in einer Zeit-Frequenz-Amplitudendomäne;
**dadurch gekennzeichnet, dass** die Vorrichtung zusätzlich:
eine Methode zum Ansatz eines psycho-akustischen Modells an das akustische Sensorsignal in der Zeit-Frequenz-Amplitudendomäne umfasst; und
ein Wiedergabegerät zur Darstellung des akustischen Sensorsignals im Bereich Zeit - wahrgenommene Frequenz - wahrgenommene Lautheit enthält.

5. Eine Vorrichtung gemäß Anspruch 4, **dadurch gekennzeichnet, dass** sie zusätzlich eine Vorrichtung zur Simulierung der Periodizitätswahrnehmung enthält.

6. Eine Vorrichtung gemäß Anspruch 4 oder 5, **dadurch gekennzeichnet, dass** sie zusätzlich eine Vorrichtung für die automatische Interpretation der Darstellung des akustischen Sensorsignals im Bereich Zeit - wahrgenommene Frequenz - wahrgenommene Lautheit enthält.

7. Ein System für die kardio-akustische Signalanalyse, das sich aus der Vorrichtung gemäß Ansprüchen 4 bis 6 zusammensetzt und zusätzlich:
ein Methode für die Aufnahme eines akustischen Sensorsignals enthält, das Herztöne darstellt; und
eine Methode für die Übertragung des akustischen Sensorsignals zum Eingabegerät enthält.

8. Ein System gemäß Anspruch 7, **dadurch gekennzeichnet, dass** die Methode für die Übertragung des akustischen Sensorsignals die Fernübertragung ermöglicht, wenn das Eingabegerät über eine Funktion für den Fernempfang des akustischen Sensorsignals verfügt.

## Revendications

1. Une méthode pour des signaux cardio-acoustiques, comprenant les opérations suivantes:
réception d'un signal d'un capteur acoustique représentatif des bruits du coeur ;
représentation du signal de capteur acoustique dans un domaine temps / fréquence / amplitude ;
**caractérisée par le fait que** la méthode comporte également l'application d'un modèle psycho-acoustique au signal du capteur acoustique dans un domaine temps / fréquence perceptive / volume sonore, afin de produire une représentation du signal du capteur acoustique dans un domaine de temps / fréquence perceptive / volume sonore.

2. Une méthode selon la revendication 1 **caractérisée par le fait que** cette méthode comporte également la simulation de la perception de la périodicité.

3. Une méthode selon la revendication 1 ou 2 **caractérisée par le fait que** cette méthode comporte également l'interprétation automatique de la représentation du signal du capteur acoustique dans un domaine de temps / fréquence perceptive / volume sonore.

4. Un dispositif pour l'analyse de signaux cardio-acoustiques, comprenant :
un dispositif d'entrée pour la réception d'un signal d'un capteur acoustique représentatif des bruits du coeur ;
un dispositif de représentation du signal de capteur acoustique dans un domaine temps / fréquence / amplitude ;
**caractérisé par le fait que** ce dispositif comprend également :
Un dispositif pour l'application d'un modèle psycho-acoustique au signal du capteur acoustique dans un domaine temps / fréquence perceptive / volume sonore ;
Un dispositif de sortie pour la présentation du signal du capteur acoustique dans un domaine de temps / fréquence perceptive / volume sonore.

5. Un dispositif selon la revendication 4 **caractérisé par le fait que** ce dispositif comporte également la simulation de la perception de la périodicité.

6. Un dispositif selon la revendication 4 ou 5 **caractérisé par le fait que** ce dispositif comporte également un dispositif pour l'interprétation automatique de la représentation du signal du capteur acoustique dans un domaine de temps / fréquence perceptive / volume sonore.

7. Un système d'analyse de signaux cadio-acoustiques, comprenant le dispositif conforme à une quelconque des revendications 4 à 6, comprenant également :
un dispositif de collecte des signaux d'un capteur acoustique représentatif des bruits du coeur ; et
un dispositif pour la transmission du signal du capteur acoustique au dispositif d'entrée.

8. Un système selon la revendication 7 **caractérisé par le fait que** le dispositif pour la transmission du signal du capteur acoustique fonctionne en transmission à distance, dans le cadre duquel le dispositif d'entrée assure la réception à distance du signal du capteur acoustique.
